# EUROPEAN PATENT APPLICATION

(11) **EP 2 168 534 A1**
(43) Date of publication of application: **31.03.2010**
(21) Application number: 09012017.1
(22) Date of filing: 22.09.2009
(51) Int. Cl.: A61F 2/16

(54) **Intraocular lens**

(30) Priority: 30.09.2008 JP 2008253884; 27.08.2009 JP 2009197248
(71) Applicant: Mikawa, Yoichi, Tokushima-shi Tokushima-ken (JP)
(72) Inventor: Mikawa, Yoichi, Tokushima-shi Tokushima-ken (JP)
(74) Representative: Lins, Edgar

(57) **Abstract**

An intraocular lens (10) to be fixed in an eyeball has an optical pupil (14) to provide a pinhole effect at the front center of the lens (10). The optical pupil (14) is constituted by a limitative pattern (13) that is partly formed in the front peripheral center of the lens (10) to limit part of an optical path.

## Description

The present invention relates to an intraocular lens fixed in an eyeball in use.

Recently, intraocular lenses (IOL) have been in practical use. The intraocular lenses are classified in accordance with the insertion position: an intraocular lens inserted to replace a crystalline lens, an intraocular lens inserted for compensation in front of an existing lens, a phakic intraocular lens (intraocular contact lens) attached (implanted) in front of a crystalline lens, etc.

Heretofore, as an intraocular lens to be inserted and fixed in an eyeball in the case of surgery for a cataract or presbyopia, a single-focus lens or a multifocal lens of a refraction type, a diffraction type, or a combination of the refraction type and the diffraction type is used.

However, the conventional single-focus lens does not necessarily ensure that sufficient effects of visual correction for both farsightedness and nearsightedness are obtained.

On the other hand, in the field of a contact lens that is attached onto the cornea (the outside of the eyeball) of the eyeball of a human being and used in an unfixed condition, a bifocal lens, a multifocal lens including an region where power naturally changes in a borderless manner, etc. have heretofore been in practical use. Moreover, in connection with the contact lens used in an unfixed condition, the idea of utilizing a pinhole effect to provide visual correction for both farsightedness and nearsightedness has been proposed in, for example, Jpn. Pat. Appln. KOKAI Publication No. 11-242191, Jpn. Pat. Appln. KOKAI Publication No. 8-29740, Japanese Patent No. 671520, Jpn. Pat. Appln. KOKAI Publication No. 2-134612, and PCT National Publication No. 9-502542. Each of the contact lenses in these documents has, in its front center, a pinhole portion for providing the pinhole effect.

However, as is well known, the contact lens that is attached onto the cornea and used in an unfixed condition moves off its attachment position due to, for example, the movement of the eyeball. Thus, the position of the pinhole portion relative to the eyeball is unstable, and the pinhole effect is not obtained stably and reliably. For such reasons, there is a difficulty in terms of practicality in using the pinhole effect for the contact lens that is attached onto the cornea.

The present invention has been made to solve the problems of the conventional intraocular lenses described above, and is directed to provide an intraocular lens that enables the pinhole effect to be obtained stably and reliably.

An intraocular lens according to the present invention has an optical pupil to provide a pinhole effect at the front center of the lens. The optical pupil is constituted by a limitative pattern that is partly formed in the front peripheral center of the lens to limit part of an optical path.

According to the present invention, there is provided an intraocular lens that enables the pinhole effect to be obtained stably and reliably. Consequently, visual correction for both farsightedness and nearsightedness and visual correction for residual astigmatism after ocular surgery are achieved stably and reliably. Moreover, a practical visual field and brightness are ensured by ensuring the amount of incident light.

The invention can be more fully understood from the following detailed description when taken in conjunction with the accompanying drawings, in which:
FIG. 1A is a perspective view schematically showing an intraocular lens according to the present invention;
FIG. 1B is a front view of a lens body of the intraocular lens shown in FIG. 1A;
FIG. 2 is a perspective view schematically showing another intraocular lens according to the present invention;
FIG. 3 is a front view schematically showing a light shielding pattern in Example 2 of the present invention;
FIG. 4 is a front view schematically showing a light shielding pattern in Example 3 of the present invention;
FIG. 5 is a front view schematically showing a light shielding pattern in Example 4 of the present invention;
FIG. 6 is a front view schematically showing a light shielding pattern in Example 5 of the present invention;
FIG. 7 is a front view schematically showing a light shielding pattern in Example 6 of the present invention;
FIG. 8 is a front view schematically showing a light shielding pattern in Example 7 of the present invention;
FIG. 9 is a front view schematically showing a light shielding pattern in Example 8 of the present invention;
FIG. 10 is a front view schematically showing a light shielding pattern in Example 9 of the present invention; and
FIG. 11 is a front view schematically showing a light shielding pattern in Example 10 of the present invention.

Embodiments of the present invention will hereinafter be described with reference to the drawings. In this description, like reference numbers are assigned to like parts throughout the drawings.

### <First Embodiment>

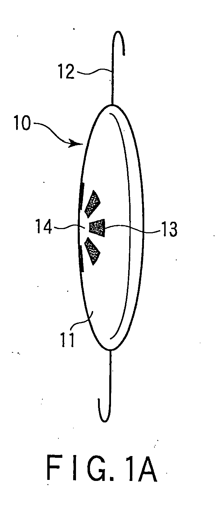

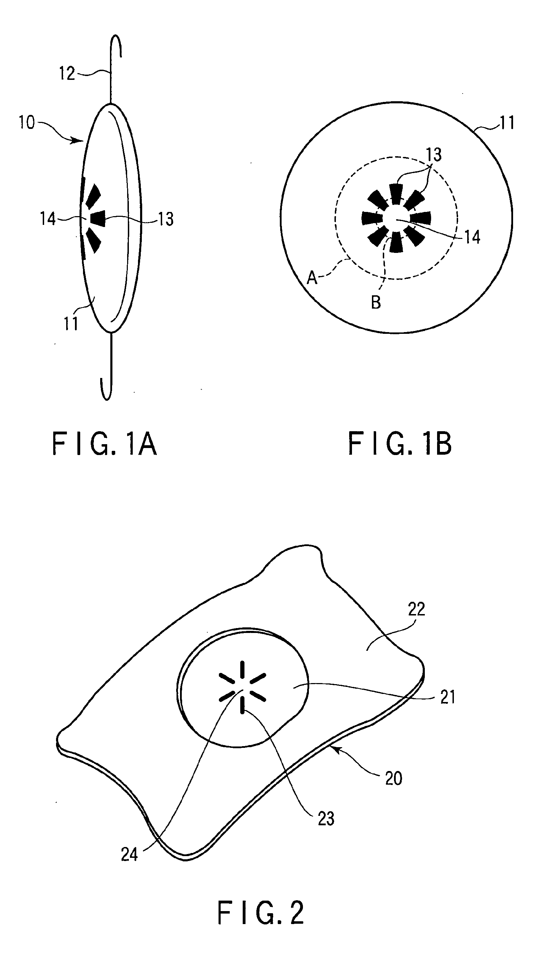

FIG. 1A is a perspective view schematically showing an intraocular lens according to a first embodiment of the present invention. As shown in FIG. 1A, an intraocular lens 10 includes an intraocular lens body 11 made of a transparent material, and a holder 12 for holding and fixing the intraocular lens body 11 in an eyeball. In the intraocular lens body 11, a limitative pattern for limiting part of an optical path (light input) is partly formed, so that an optical pupil 14 for providing a pinhole effect is formed in the front center. As described later, considering a practically proper aperture ratio, it is possible to obtain various shapes of the limitative pattern, various ranges of optical path limitation by the limitative pattern, and various amounts of light input limitation by the limitative pattern.

FIG. 1B is a front view of the intraocular lens body shown in FIG. 1A. In the intraocular lens body 11, the limitative pattern (a light shielding pattern 13 in this example) for forming the optical pupil 14 in the front center is formed in the front peripheral center (the periphery of the front center) of the intraocular lens body 11.

The intraocular lens body 11 has only to be a transparent lens with or without power. The intraocular lens body 11 may have any shape. The material of the intraocular lens body 11 has only to be nonpoisonous to the eye and may be colored. Intraocular lenses of existing shapes and materials (PMMA, an acrylic resin, a silicon resin) can be used. The intraocular lens body 11 functions as an artificial crystalline lens to be held and fixed in the eyeball in place of a crystalline lens removed by a cataract operation.

The overall size of the light shielding pattern 13 is smaller than a size of the pupil formed by the iris of the eyeball in night vision. Here, one example of the outer edge of the pupil in night vision is indicated by A in FIG. 1B. Moreover, the size of the optical pupil 14 is smaller than a size of the pupil formed by the iris of the eyeball in the case of a normal light input (in clear vision). Here, one example of the outer edge of the pupil in clear vision is indicated by B in FIG. 1B.

The light shielding pattern 13 is desirably formed at a position concentric with the center as viewed from the front of the intraocular lens body 11. The overall shape of the light shielding pattern 13 can be set to any shape, for example, a shape continuously or discontinuously formed in the direction of the outer periphery of the center. The optical pupil 14 formed in the center of the light shielding pattern 13 is not limited to a circular shape, and may be a polygonal shape such as a quadrangular, hexagonal or octagonal shape.

A material that is nonpoisonous to the eye is used for the light shielding pattern 13. The light shielding pattern 13 can be formed of, for example, a thin film. The light shielding pattern 13 is not exclusively formed on the surface of the intraocular lens body 11. Any position can be selected to form the light shielding pattern 13; for example, a superposition position within the lens body in the case of a superposition structure.

The degree of light shielding (optical path limitation) by the light shielding pattern 13 may be constant or partially changed. For example, the light shielding pattern 13 may be formed by being intermittently arranged in the length direction in a ring region concentric with the front center. Moreover, a dimming material may be used to form the light shielding pattern 13 to provide a dimmer function of partially or entirely changing the degree of light shielding or the degree of coloring in accordance with the amount of input light.

The intraocular lens 10 of the present embodiment described above is inserted into the eyeball of a human being, and the intraocular lens body 11 is replaced with a crystalline lens. The pinhole effect is utilized for the intraocular lens used in an intraocularly fixed condition, so that the following effects can be obtained. As the relative position of the optical pupil in the center of the lens front surface in the eyeball is stable, the pinhole effect is obtained stably and reliably. The depth of focus is increased and the depth of visual recognition is increased owing to the pinhole effect, so that the effect of visual correction for both farsightedness and nearsightedness is obtained stably and reliably, and an age-related reduction in adjustment ability can be relieved. Moreover, even if there is residual astigmatism (astigmatism associated with the distortion of the cornea or the eyeball itself) after ocular surgery, diplopia is not easily caused in contrast with a multifocal lens having a conventional structure and the effect of visual correction can be obtained owing to the pinhole effect. Another advantage of the intraocular lens 10 of the present embodiment is that a halo or glare is not easily caused in contrast with a multifocal lens having a conventional structure.

In addition, the size of the optical pupil 14 formed by the light shielding pattern 13 is smaller than a size of the pupil formed by the iris of the eyeball in clear vision, and the size of the light shielding pattern 13 is smaller than a size of the pupil formed by the iris of the eyeball in night vision, so that light input to a transparent region of the intraocular lens body 11 between the outer edge of the pupil and the outer edge of the light shielding pattern 13 is effective. Thus, a practical intraocular lens is obtained in which a practical visual field and brightness can be ensured by ensuring the amount of incident light.

### <Second Embodiment>

FIG. 2 is a perspective view schematically showing an intraocular lens according to a second embodiment of the present invention. The intraocular lens according to the second embodiment is a contact lens for intraocular attachment, which is inserted into an eyeball, e.g., of a human being, attached (implanted) in front of a crystalline lens in the eyeball, and used in an intraocularly fixed condition. The intraocular lens 20 includes a lens body portion 21 made of a transparent material and attached in front of the crystalline lens, and a peripheral portion 22 surrounding the lens body portion 21. The lens body portion 21 and the peripheral portion 22 may be integrally formed. The lens body portion 21 and the peripheral portion 22 may be also formed as separated members and then joined to each other. The peripheral portion 22 functions as a holder for holding and fixing the lens body portion 21 in the eyeball.

In the lens body portion 21, a limitative pattern (a light shielding pattern 23 in this example) is formed in the front peripheral center (the periphery of the front center) to form an optical pupil 24 for providing the pinhole effect in the front center. In this example, the light shielding pattern 23 is formed in the front peripheral center alone.

The lens body portion 21 has only to be a transparent lens with or without power. The lens body portion 21 may have any shape. The material of the lens body portion 21 has only to be nonpoisonous to the eye and may be colored. Existing shapes and materials (PMMA, a silicon resin, etc.) can be used.

The overall area of the light shielding pattern 23 is smaller than a size of the pupil formed by the iris of the eyeball in night vision. The size of the optical pupil 24 is smaller than a size of the pupil formed by the iris of the eyeball in clear vision.

The light shielding pattern 23 is desirably formed at a position concentric with the center when viewed in front of the lens body portion 21. The overall shape of the light shielding pattern 23 can be set to any shape, for example, a shape continuously or discontinuously formed in the direction of the outer periphery of the center. The optical pupil 24 formed in the center of the light shielding pattern 23 is not limited to a circular shape, and may be a polygonal shape such as a quadrangular, hexagonal or octagonal shape.

A material that is nonpoisonous to the eye is used for the light shielding pattern 23. The light shielding pattern 23 can be formed of, for example, a thin film. The light shielding pattern 23 is not exclusively formed on the surface of the lens body portion 21. Any position can be selected to form the light shielding pattern 23; for example, a superposition position within the lens body portion in the case of a superposition structure.

The degree of light shielding (optical path limitation) by the light shielding pattern 23 may be constant or partially changed. For example, the light shielding pattern 23 may be formed by being intermittently arranged in the length direction in a ring region concentric with the front center. Moreover, a dimming material may be used to form the light shielding pattern 23 to provide a dimmer function of partially or entirely changing the degree of light shielding or the degree of coloring in accordance with the amount of input light.

In addition, a pattern (e.g., a decoration pattern in the shape of a large pupil) other than the light shielding pattern may be formed around the light shielding pattern 23.

According to the second embodiment described above, the pinhole effect is utilized for the intraocular lens, which is attached (implanted) in front of the crystalline lens in the eyeball with the crystalline lens used as it is, and is used in an intraocularly fixed condition. Consequently, so that the effects similar to the effects in the first embodiment described above are obtained.

### [Example 1]

FIG. 1B is a front view schematically showing a light shielding pattern in Example 1 of the present invention. This light shielding pattern 13 has a plurality of (e.g., eight) thin radial patterns discontinuously formed at a small pitch in a circular ring region at the front peripheral center of an intraocular lens body 11. The overall size (in this example, the diameter of the circular ring region where the light shielding pattern is formed) of the light shielding pattern 13 is set at a proper value ranging from 5 mm to 6 mm, which is smaller than a size of the pupil formed by the iris of the eyeball in night vision. An optical pupil 14 in the center of the light shielding pattern is substantially circular, and its diameter is set at a proper value ranging from 0.2 mm to 2.5 mm, which is smaller than a size of the pupil formed by the iris of the eyeball in clear vision.

According to Example 1, the size of the light shielding pattern 13 is smaller than the size of the pupil in night vision. The size of the optical pupil 14 is smaller than a size of the pupil in clear vision. Thus, it is possible to obtain a practical intraocular lens that enables visual correction for both farsightedness and nearsightedness by utilizing the pinhole effect and that can ensure a practical visual field and brightness. Moreover, since the radial patterns are discontinuously formed as the light shielding pattern 13, it is possible to secure an optical path where a posterior capsule is cut open by laser after the insertion of the intraocular lens.

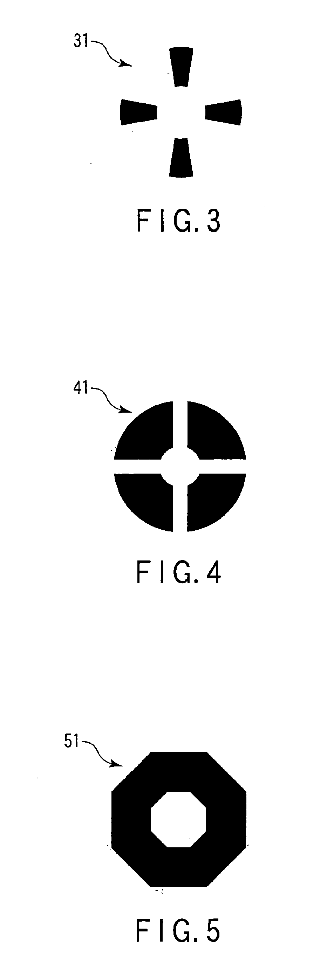

In addition, the light shielding pattern 13 may be formed to have a dimmer function, so that when the amount of input light is small, the pattern may be changed to a radial pattern with a great pitch as shown in FIG. 3 in Example 2 described later to increase brightness.

### [Example 2]

FIG. 3 is a front view schematically showing a light shielding pattern in Example 2 of the present invention. This light shielding pattern 31 has a plurality of (e.g., four) thin radial patterns discontinuously formed at a great pitch in a circular ring region at the front peripheral center of a lens body. An optical pupil in the center of the light shielding pattern is substantially circular. The size of the light shielding pattern 31 and the size of the optical pupil are similar to the sizes in Example 1 described above. According to Example 2, the effects similar to the effects in Example 1 described above are obtained.

### [Example 3]

FIG. 4 is a front view schematically showing a light shielding pattern in Example 3 of the present invention. This light shielding pattern 41 has a plurality of (e.g., four) thick ring-shaped patterns discontinuously formed in a circular ring region at the front peripheral center of a lens body. An optical pupil in the center of the light shielding pattern is substantially circular. The size of the light shielding pattern 41 and the size of the optical pupil are similar to the sizes in Example 1 described above.

According to Example 3, the effects similar to the effects in Example 1 described above are obtained. Moreover, since the thick patterns are formed as the light shielding pattern 41, the pinhole effect is great, and great visual correction effects can be expected.

### [Example 4]

FIG. 5 is a front view schematically showing a light shielding pattern in Example 4 of the present invention. This light shielding pattern 51 has a thick pattern continuously formed in an octagonal ring region at the front peripheral center of a lens body. An optical pupil in the center of the light shielding pattern is octagonal. The size of the light shielding pattern 51 and the size of the optical pupil are similar to the sizes in Example 1 described above.

According to Example 4, it is possible to obtain a practical intraocular lens that enables visual correction for both farsightedness and nearsightedness by utilizing the pinhole effect and that can ensure a practical visual field and brightness, as in Example 1 described above. Moreover, since the thick pattern is continuously formed as the light shielding pattern 51, the pinhole effect is great, and great visual correction effects can be expected. In this case, the optical pupil is octagonal, so that the pinhole effect different from that in Example 1 is obtained, and a proper visual correction effect adapted to an individual difference can be expected.

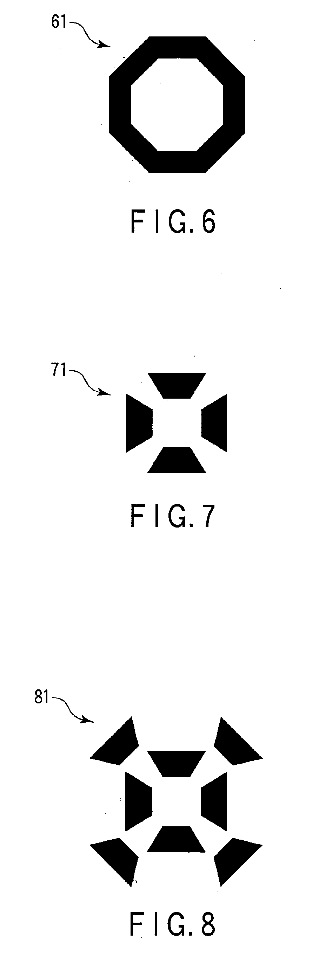

In addition, the light shielding pattern 51 may be formed to have a dimmer function, so that when the amount of input light is small, the pattern may be changed to a thin pattern as shown in FIG. 6 in Example 5 described later to increase brightness.

### [Example 5]

FIG. 6 is a front view schematically showing a light shielding pattern in Example 5 of the present invention. This light shielding pattern 61 has a thin pattern continuously formed in an octagonal ring region at the front peripheral center of a lens body. An optical pupil in the center of the light shielding pattern is octagonal. The size of the light shielding pattern 61 and the size of the optical pupil are similar to the sizes in Example 1 described above.

According to Example 5, it is possible to obtain a practical intraocular lens that enables visual correction for both farsightedness and nearsightedness by utilizing the pinhole effect and that can ensure a practical visual field and brightness, as in Example 4 described above. Moreover, since the pattern is continuously formed as the light shielding pattern 61, the pinhole effect is great, and great visual correction effects can be expected. In this case, the optical pupil is octagonal, so that the pinhole effect different from that in Example 1 is obtained, and a proper visual correction effect adapted to an individual difference can be expected.

### [Example 6]

FIG. 7 is a front view schematically showing a light shielding pattern in Example 6 of the present invention. This light shielding pattern 71 has a plurality of (e.g., four) thick patterns discontinuously formed in a quadrangular ring region at the front peripheral center of a lens body. An optical pupil in the center of the light shielding pattern is substantially quadrangular. The size of the light shielding pattern 71 and the size of the optical pupil are similar to the sizes in Example 1 described above.

According to Example 6, the effects similar to the effects in Example 1 described above are obtained. In this case, the optical pupil is quadrangular, so that the pinhole effect different from that in Example 1 is obtained, and a proper visual correction effect adapted to an individual difference can be expected.

### [Example 7]

FIG. 8 is a front view schematically showing a light shielding pattern in Example 7 of the present invention. In this light shielding pattern 81, a thin pattern is additionally formed discontinuously in an octagonal ring region to face the outside of each corner of the light shielding pattern shown in Example 6. An optical pupil in the center of the light shielding pattern is substantially quadrangular. The size of the light shielding pattern 81 and the size of the optical pupil are similar to the sizes in Example 1 described above.

According to Example 7, the effects similar to the effects in Example 1 described above are obtained. In this case, if at least the size of the inner ring region in the light shielding pattern 81 is formed to be smaller than the size of the pupil in night vision, light input to a transparent region of a lens body between the outer edge of the pupil and the outer edge of the inner ring region is effective.

In addition, the light shielding pattern 61 in Example 7 may be formed to have a dimmer function, so that when the amount of input light is small, the pattern may be changed to the quadrangular ring pattern as shown in FIG. 7 in Example 6 described above to increase brightness.

### [Example 8]

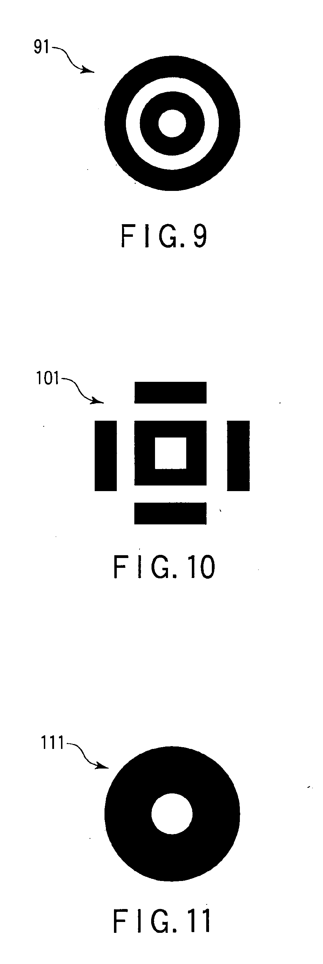

FIG. 9 is a front view schematically showing a light shielding pattern in Example 8 of the present invention. This light shielding pattern 91 has a thin pattern continuously formed in a double circular ring region at the front peripheral center of a lens body. An optical pupil in the center of the light shielding pattern is circular. The size of the light shielding pattern 91 and the size of the optical pupil are similar to the sizes in Example 1 described above.

According to Example 8, it is possible to obtain a practical intraocular lens that enables visual correction for both farsightedness and nearsightedness by utilizing the pinhole effect and that can ensure a practical visual field and brightness, substantially as in Example 1 described above. In this case, if at least the size of the inner ring region in the light shielding pattern 91 is formed to be smaller than the size of the pupil in night vision, light input to a transparent region of a lens body between the outer edge of the pupil and the outer edge of the inner ring region is effective.

Moreover, since the pattern is continuously formed as the light shielding pattern 91, the pinhole effect is great, and great visual correction effects can be expected.

### [Example 9]

FIG. 10 is a front view schematically showing a light shielding pattern in Example 9 of the present invention. This light shielding pattern 101 has a thin pattern continuously formed in a quadrangular ring region at the front peripheral center of a lens body, and further has a thin linear pattern formed discontinuously in a quadrangular ring region to face the outside of each side of the former pattern. An optical pupil in the center of the light shielding pattern is quadrangular. The size of the light shielding pattern 101 and the size of the optical pupil are similar to the sizes in Example 1 described above.

According to Example 9, it is possible to obtain a practical intraocular lens that enables visual correction for both farsightedness and nearsightedness by utilizing the pinhole effect and that can ensure a practical visual field and brightness, substantially as in Example 1 described above. In this case, if at least the size of the inner ring region in the light shielding pattern 101 is formed to be smaller than the size of the pupil in night vision, light input to a transparent region of a lens body between the outer edge of the pupil and the outer edge of the inner ring region is effective.

Moreover, since the pattern is continuously formed as the light shielding pattern 101, the pinhole effect is great, and great visual correction effects can be expected. In this case, the optical pupil is quadrangular, so that the pinhole effect different from that in Example 1 is obtained, and a proper visual correction effect adapted to an individual difference can be expected.

### [Example 10]

FIG. 11 is a front view schematically showing a light shielding pattern in Example 10 of the present invention. This light shielding pattern 111 has a thick pattern continuously formed in a circular ring region at the front peripheral center of a lens body. An optical pupil in the center of the light shielding pattern is circular. The size of the light shielding pattern 111 and the size of the optical pupil are similar to the sizes in Example 1 described above.

According to Example 10, it is possible to obtain a practical intraocular lens that enables visual correction for both farsightedness and nearsightedness by utilizing the pinhole effect and that can ensure a practical visual field and brightness. Moreover, since the thick pattern is continuously formed as the light shielding pattern 111, the pinhole effect is great, and great visual correction effects can be expected.

In addition, the light shielding pattern 111 in Example 10 may be formed to have a dimmer function, so that when the amount of input light is small, the pattern may be changed to the double circular ring region as shown in FIG. 9 in Example 8 described above or to the discontinuous circular ring pattern as shown in FIG. 4 in Example 3 described above to increase brightness.

It is to be noted that the present invention is not limited to the embodiments and Examples described above. It goes without saying that various modifications can be made within the scope of the invention defined in claims and that such modifications fall within the scope of the invention. For example, the present invention is also applicable to an intraocular lens for animals other than human beings.

## Claims

1. An intraocular lens (10; 20) to be fixed in an eyeball, **characterized by** having an optical pupil (14; 24) to provide a pinhole effect at the front center of the lens (10; 20), the optical pupil (14; 24) being constituted by a limitative pattern (13; 23; 31; 41; 51; 61; 71; 81; 91; 101; 111) that is partly formed in the front peripheral center of the lens (10; 20) to limit part of an optical path.

2. The intraocular lens (10) according to claim 1, **characterized by** comprising:
an intraocular lens body (11) in which the optical pupil (14) is formed; and
a holder (12) to hold and fix the intraocular lens body (11) in the eyeball.

3. The intraocular lens (20) according to claim 1, **characterized by** comprising:
a lens body portion (21) in which the optical pupil (24) is formed, the lens body portion (21) being attached in front of a crystalline lens in the eyeball; and
a peripheral portion (22) surrounding the lens body portion (21), the peripheral portion (22) functioning as a holder to hold and fix the lens body portion (21) in the eyeball.
